(19) **Europäisches Patentamt / European Patent Office / Office européen des brevets**

(11) **EP 2 687 263 B1**

(12) **EUROPEAN PATENT SPECIFICATION**

(45) Date of publication and mention of the grant of the patent:
**19.10.2016 Bulletin 2016/42**

(51) Int Cl.:
*A61K 36/54* (2006.01)    *A61P 7/02* (2006.01)

(21) Application number: **13188806.7**

(22) Date of filing: **19.11.2010**

(54) **Composition comprising the extracts of Lindera Obtusiloba as effective ingredient for prevention and treatment of thrombosis**

Zusammensetzung mit Extrakten aus Lindera obtusiloba als wirksamer Bestandteil zur Prävention und Behandlung von Thrombose

Composition comprenant des extraits de Lindera Obtusiloba comme ingrédient efficace pour la prévention et le traitement de la thrombose

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2009 KR 20090112484**

(43) Date of publication of application:
**22.01.2014 Bulletin 2014/04**

(62) Document number(s) of the earlier application(s) in accordance with Art. 76 EPC:
**10831803.1 / 2 510 933**

(73) Proprietors:
- **Han Wha Pharma Co., Ltd.**
  **Chuncheon**
  **Gangwon-do**
  **200-921 (KR)**
- **Yang Ji Chemical Co., Ltd.**
  **Ansan, Gyeonggi-do 425-833 (KR)**

(72) Inventors:
- **Oak, Min-Ho**
  **435-710 Gyeonggi-do (KR)**
- **Lee, Jung-Ok**
  **440-729 Gyeonggi-do (KR)**
- **Kang, So-Hee**
  **Gyeonggi-do, 441-853 (KR)**
- **Sohn, Jung-Duk**
  **430-705 Gyeonggi-do (KR)**
- **Kim, Jong-Hoon**
  **431-708 Gyeonggi-do (KR)**
- **Lim, Jee Woong**
  **463-779 Gyeonggi-do (KR)**
- **Na, Yongho**
  **426-735 Gyeonggi-do (KR)**
- **Oh, Youna**
  **134-782 Seoul (KR)**
- **Lee, Seung-Woo**
  **135-090 Seoul (KR)**

(74) Representative: **Ter Meer Steinmeister & Partner Patentanwälte mbB**
**Nymphenburger Straße 4**
**80335 München (DE)**

(56) References cited:
**WO-A2-2009/091120**

- **DATABASE TCM [Online] SIPO; 27 February 2008 (2008-02-27), Zhang Lin et al.: "Preparation method and its use of Chinese medicine extract for treating cardiovascular and cerebrovascular diseases", XP002692788, Database accession no. CN-200710070623-A & CN 101 129 466 A (UNIV ZHEJIANG [CN] UNIV ZHEJIANG) 27 February 2008 (2008-02-27)**
- **KWON H C ET AL: "New Cytotoxic butanolides from Lindera obtusiloba Blume", CHEMICAL AND PHARMACEUTICAL BULLETIN, vol. 48, no. 5, 1 January 2000 (2000-01-01), pages 614-616, XP003010892, PHARMACEUTICAL SOCIETY OF JAPAN, JP ISSN: 0009-2363**

- **DATABASE WPI Week 200666 Thomson Scientific, London, GB; AN 2006-627598 XP002692829, & CN 1 739 690 A (WANG H) 1 March 2006 (2006-03-01)**

**Description**

**[Technical Field]**

The present invention relates to compositions for improving blood circulation, comprising an extract of *Lindera obtusiloba* as an active ingredient, and more particularly, to a pharmaceutical composition and a health functional food for preventing and treating thrombotic disorders by improving blood circulation, comprising an extract of *Lindera obtusiloba* as an active ingredient.

**[Background Art]**

Humans have a biological system for healing wound and preventing blood loss, which consists of regulation and balance of a complicated process of coagulation of platelets and plasma, fibrinolysis, and inhibition of coagulation. However, the breakdown of this equilibrium state caused by a number of factors can lead to disturbances of blood flow, and subsequently cause thrombotic disorders and tissue functional disorders.

Thrombosis is the formation of a blood clot or thrombus inside a blood vessel, obstructing the flow of blood. When a blood vessel is injured, the blood vessel contracts, and platelets adhere to exposed collagen fibers of a wound and release vasoconstrictor substances such as serotonin, adenosine diphosphate (ADP), and thromboxane A2. ADP causes more platelets to adhere, and thromboxane A2 promotes platelet aggregation, and the blood vessel contracts. After primary hemostasis like this, blood coagulation occurs. Fibrin formed after hemostasis is dissolved by fibrinolysis, and tissue-type plasminogen activator (t-PA) and urokinase (UK) convert plasminogen to plasmin, thus allowing fibrin to breakdown. Normally, blood coagulation and fibrinolysis are balanced, however, when blood coagulation and fibrinolysis are not balanced, fiber lump is deposited in a blood vessel to occur thrombosis, obstructing blood circulation (Nesheim M., Chest., 2003 Sep;124:33S-9S).

Thrombosis can lead to atherothrombotic diseases, phlebothrombosis, hepatic portal vein thrombosis, pulmonary thromboembolism, chronic limb ischemia, varicose vein, deep vein thrombosis diseases, angina pectoris, cerebral infarction, cerebral hemorrhage, *etc.* It can also lead to infection or damage of blood vessels, postoperative complications, coagulative diseases, *etc.*

Antiplatelet agents, anticoagulants, thrombolytic agents for treating formed thrombus, *etc.* are currently used for the prevention and treatment of thrombotic diseases. A representative antiplatelet agent, aspirin is known to have excellent effect, but cause side effects such as upper gastrointestinal tract bleeding, peptic ulcers, *etc.* Drugs used for other anticoagulants or therapeutic agents for hyperlipidemias are mostly impossible to administer orally, have low selectivity for thrombus, and show various side effects of long-term administration, such as hemolytic phenomenon, immune responses, fever, allergies, *etc.* Despite such side effects and incomplete effectiveness, prices of available therapeutic agents are expensive. So, the problem that patients have difficulty to use these therapeutic agents easily came to the fore. Accordingly, development of therapeutic agents with high selectivity for inhibition of thrombus formation, such as activation of platelets, inhibition of coagulation, thrombolytic activity, *etc.,* and minimum side effects is urgently needed.

*Lindera obtusiloba* is a deciduous shrub in the Lauraceae family. Trees grow up to approximately 2 to 3 m tall, flowering season is around March to April, and fruiting season is September. Oil pressed from fruits used for hair oil for women. When breaking off a leaf or branch, it smells of ginger, and so, it is called a ginger plant, called also *Slyrax obassia* or *Benzoin obtusiloboum.* It has efficacies in invigorating blood, soothing sinews, and alleviating edema. It is known to treat stasis blood, swelling and pain caused by a bruise. It is usually crushed to apply to wounds. It is used mainly for stomachache, and can be used also as a fever reducer and cough medicine. Leaves and sprouts are boiled down and taken like teas as a fever reducer and cough medicine in folk medicine (An Illustrated Guide to Korean Flora, Lee Changbok, 1980; An Illustrated Guide to Korean Folk Drugs, Park Jonghee, 2005, Unabridged dictionary of native herbal drugs).

With regard to research on components of *Lindera Obtusiloba*, Kwon Hakcheol, et al. (Archives of Pharmacal Research, 22, p. 417-422, 1999) isolated actifolin, pluviatiolol, 5,6-dihydromataireinol, (+)-syringaresinol, 9-O-trans-feruloyl-5,5-dimethoxylariciresinol, *etc.* The efficacies of the respective isolated components are reported to include cancer cell-killing action, anti-inflammatory action, *etc.* (Planta Medica, 69, 610-616, 2003; Archives of Pharmacal Research, 22, p. 417-422, 1999).

However, no disclosure or teaching is given in the above mentioned literatures, on that extracts of *Lindera obtusiloba* have effects of inhibition of platelet aggregation, inhibition of thrombus formation, treatment and prevention of thrombotic disorders.

**[Disclosure]**

**[Technical Problem]**

The present inventors have performed research on agents for improving blood circulation from numerous plant extracts, and found that extracts of *Lindera obtusiloba* had excellent efficacies in inhibiting platelet aggregation, inhibiting and preventing thrombus formation *in vivo,* thereby leading to completion of the present invention.

The present invention was devised based on the above findings, and provides a pharmaceutical composition for preventing and treating thrombotic disorders by improving blood circulation, the composition comprising an extract of *Lindera obtusiloba* as an active ingredient.

The present invention also provides a health functional food for preventing thrombotic disorders, comprising the extract of *Lindera abtusiloba* as an active ingredient.

**[Technical Solution]**

In accordance with an exemplary embodiment, a composition for preventing and treating thrombotic disorders by improving blood circulation of the present invention comprises an extract of *Lindera obtusiloba* as an active ingredient in order to achieve the above described objects.

The extract of *Lindera obtusiloba* is extracted from *Lindera obtusiloba* leaves.

The extract of *Lindera obtusiloba* may be extracted with a solvent selected from the group consisting of water, a lower alcohol of $C_1$ to $C_5$, and a mixture thereof.

The lower alcohol of $C_1$ to $C_5$ may be methanol, ethanol, or butanol.

The extract of *Lindera obtusiloba* may be extracted with from about 30 to about 95 % by weight of an aqueous ethanol solution.

The extract of *Lindera obtusiloba* may be extracted at from about 30 to about 95°C.

The extract of *Lindera obtusiloba* may be extracted with a solvent selected from the group consisting of water, a lower alcohol of $C$, to $C_3$, and a mixture thereof, and then extracted again with butanol.

The extract of *Lindera obtusiloba* may be prepared by obtaining a crude extract through an extraction with a solvent selected from the group consisting of water, a lower alcohol of $C_1$ to $C_3$, and a mixture thereof and concentration, suspending the crude extract into water, and extracting the suspended crude extract again in the order of hexane, ethylacetate, and butanol.

The crude extract and water may be mixed and suspended in a volume ratio of the crude extract: water of 1 : 5 to 1 : 25.

The extract of *Lindera obtusiloba* may have platelet aggregation-inhibitory activity.

The extract of *Lindera obtusiloba* may have *in vivo* thrombus formation-inhibitory activity.

The thrombotic disorder may be selected from the group of atherothrombotic diseases, phlebothrombosis, hepatic portal vein thrombosis, pulmonary thromboembolism, chronic limb ischemia, varicose vein, deep vein thrombosis diseases, cerebral infarction, cerebral hemorrhage, postoperative blood vessel complications, and coagulative diseases.

In accordance with another exemplary embodiment, a health functional food for preventing thrombotic disorders by improving blood circulation of the present invention comprises the extract of *Lindera obtusiloba* as an active ingredient.

**[Advantageous Effects]**

As described above, the extract of *Lindera obtusiloba* of the present invention has not only excellent inhibitory effect on platelet aggregation induced by various aggregation inductions *in vitro,* but also excellent inhibitory effect on rapid thrombus formation *in vivo.* Thus, the extract of *Lindera obtusiloba* of the present invention can be useful for preventing and treating diseases caused by blood circulation disorders such as thromboembolism, *etc.*

**[Description of Drawings]**

FIG. 1 is graphs comparing inhibitory effect of the extracts according to one embodiment of the present invention on platelet aggregation when collagen was used as a platelet aggregation inducer.

FIG. 2 is graphs comparing inhibitory effect of the extracts according to one embodiment of the present invention on platelet aggregation when ADP was used as a platelet aggregation inducer.

FIG. 3 is a graph measuring cytotoxicity of *Lindera obtusiloba* extracts.

**[Mode for Invention]**

Hereinafter, preferred embodiments of the present invention will be described in detail. Many specific details, such as particular components *etc.* are shown in the following description and these have been provided only for helping the overall understanding of the present invention. It would be obvious to those skilled in the art that the present invention can be carried out without these specific details. Also, detailed descriptions of well-known functions and structures related to the present invention will be omitted so as not to unnecessarily obscure the important point of the present invention.

*Lindera obtusiloba* extracts of the present invention may be obtained as follows.

A ground part of the *Lindera obtusiloba*, more specifically, leaves and branches may be anything, but without limitation, that are collected, cultivated, or purchased, *etc.* Extraction solvent is selected from the group consisting of water, a lower alcohol of $C_1$ to $C_5$, and a mixture thereof. The present inventors washed branches and leaves of *Lindera obtusiloba* with water, removed foreign substances and salt therefrom, and dried the branches and leaves of *Lindera obtusiloba.* Extraction with water, a polar solvent of a lower alcohol of $C_1$ to $C_5$, such as methanol, ethanol, butanol, *etc.,* or a mixed solvent thereof having mix ratios of from about 1 : 0.1 to about 1 : 10, preferably an aqueous ethanol solution of from about 30 to about 95 % by weight, wherein a volume of water, the polar solvent, or the mixed solvent is from about 5 times to about 50 times, preferably from about 10 times to about 30 times of the weight of *Lindera obtusiloba* sample, at from about 50 to about 95°C, for 1 hour to 7 days is carried out two to five times, preferably 3 times repeatedly. Or, extraction with water or an aqueous lower alcohol solution, followed by re-extraction with butanol is more preferable. Next, a crude extract of *Lindera obtusiloba* is obtained by concentrating under reduced pressure and/or freeze drying the extract.

Among extracts of the present invention, non-polar solvent-soluble extracts can be obtained by suspending the crude extract in water, adding a non-polar solvent such as hexane, ethylacetate, and chloroform in an amount of about 0.1 to 100 times, preferably about 1 to 5 times of the suspension, extracting with the non-polar solvent about 1 to 10 times, preferably 2 to 5 times, and separating. Conventional fractionation may be carried out additionally (Harborne. J.B., Phytochemical methods : A guide to modern techniques of plant analysis, 3rd Ed., pp.6-7, 1998).

More preferably, a hexane fraction, an ethylacetate fraction, and an n-butanol fraction of *Lindera obtusiloba* may be obtained by stepwise solvent-fractionating the crude extract of *Lindera obtusiloba* obtained by the above process, preferably the aqueous ethanol solution extract of *Lindera obtusiloba* with organic solvents such as n-butanol, hexane, ethylacetate, *etc.* in order of increasing polarity, preferably hexane, ethylacetate, and n-butanol in order, and concentrating under reduced pressure.

The present invention provides a pharmaceutical composition for preventing and treating thrombotic disorders, comprising the extract, the crude extract, the non-polar solvent-soluble extract, or the fraction of *Lindera obtusiloba* obtained by the above preparation method, as an active ingredient.

The pharmaceutical composition for preventing and treating thrombotic disorders according to the present invention comprises 0.1 to 99 % by weight of the extract with respect to total weight of the composition.

The composition comprising the extract of *Lindera obtusiloba* of the present invention may further comprise suitable carriers, excipients, and diluents that are conventionally used for the preparation of compositions.

In pharmaceutical dosage forms, the extract of the present invention may be used as pharmaceutically acceptable salts thereof, or used alone or in combination as well as suitable assembly with other pharmaceutically active compounds.

The pharmaceutical composition comprising the extract according to the present invention may be used as oral formulations such as powders, granules, tablets, capsules, suspensions, emulsions, syrup, aerosols, *etc.,* external formulations, suppositories, and sterile injections by general methods.

Examples of carriers, excipients, and diluents that may be comprised in the composition comprising the extract include lactose, dextrose, sucrose, sorbitol, mannitol, xylitol, erythritol, maltitol, starch, acacia gum, alginate, gelatin, dibasic calcium phosphate, monobasic calcium phosphate, dibasic sodium phosphate, monobasic sodium phosphate, calcium silicate, cellulose, methylcellulose, microcrystalline cellulose, polyvinylpyrrolidone, water, methyl hydroxybenzoate, propyl hydroxybenzoate, talc, magnesium stearate, and mineral oil.

Formulations may prepared by using diluents or excipients such as fillers, extenders, binders, humectants, disintegrators, surfactants, *etc.* that are generally used.

Solid formulations for oral administration include tablets, pills, powders, granules, capsules, *etc.* and these solid formulations are prepared by mixing at least one or more excipients, for example, starch, calcium carbonate, sucrose or lactose, gelatin, microcrystalline cellulose, *etc.* with the extract. Also, lubricants such as magnesium stearate, talc, *etc.* are used in addition to simple excipients.

Liquid formulations for oral administration include suspensions, liquid for internal use, emulsions, syrups, *etc.*, and various excipients such as humectants, sweeteners, aromatics, preservatives, etc. in addition to generally-used simple diluents such as water and liquid paraffin may be included.

Formulations for parenteral administration include sterile solutions, non-aqueous solvents, suspensions, emulsions, freeze-dried formulations, and suppositories. Propylene glycol, polyethylene glycol, vegetable oil such as olive oil, and injectable ester such as ethylolate, *etc.* may be used for non-aqueous solvents and suspensions. Witepsol, macrogol, tween, cacao butter, laurin butter, glycerogelatin, *etc.* may be used for a suppository base.

The preferred administration dose of the extract of the present invention may be different depending on condition and body weight of a patient, severity of disease, drug form, administration route, and administration period, and be selected appropriately by those skilled in the art. For preferable effects, the extract of the present invention may be administered in a dose of from about 0.0001 to about 100 mg/kg body weight per day, preferably from about 0.001 to about 100 mg/kg. The administration frequency may be once a day or a few times a day. The administration dose is not intended to limit the scope of the present invention in any way.

The present invention provides a health functional food comprising the extract having preventive effect on thrombotic disorders and a sitologically acceptable dietary supplement additive.

Examples of health functional foods to which the extract of *Lindera obtusiloba* can be added include a variety of general foods, beverages, gum, teas, vitamin complexes, *etc.*

Also, the extract of *Lindera obtusiloba* may be added to foods or beverages for the purpose of preventing thrombus formation. An amount of the extract in foods or beverages may be 0.01 to 15% by weight of total food weight, and an amount of the extract in health beverages may be 0.02 to 5 g, preferably 0.3 to 1 g, based on 100 g of a health beverage composition.

The health functional beverage composition of the present invention has no particular limitation to other ingredients except that it comprises the indicated ratio of the extract as an essential ingredient, and like general beverages, it may comprise additional ingredients such as various flavoring agents or natural carbohydrates. Examples of the above described natural carbohydrates include monosaccharides such as glucose and fructose, disaccharides such as maltose and sucrose, polysaccharides, for example, general sugars such as dextrin and cyclodextrin, and sugar alcohols such as xylitol, sorbitol, erythritol, *etc.* In addition to the above described flavoring agents, natural flavoring agents such as thaumatin and stevia extracts, e.g., rebaudioside A, glycyrrhizin, *etc.*, and synthetic flavoring agents such as saccharin and aspartame may be used favorably for the flavoring agents. The ratio of the natural carbohydrate is generally about 1 to 20 g per 100 g of the composition of the present invention, preferably about 5 to 12 g.

In addition to that, the extract of the present invention may comprise various nutritional supplements, vitamins, minerals (electrolytes), synthetic and natural flavors, colorants and fillers (cheese, chocolates, *etc.*), pectic acid and its salt, alginic acid and its salt, organic acids, protective colloidal thickeners, pH regulating agents, stabilizers, preservatives, glycerin, alcohols, carbonizing agents used in carbonated drinks, *etc.* Moreover, extracts of the present invention may comprise fruit flesh for the preparation of natural fruit juices, fruit juice beverages, and vegetable beverages. These ingredients may be used alone or in combination. Although not critical, these additives are generally used in an amount from about 0.01 to about 20 parts by weight, based on 100 parts by weight of the extract of the present invention.

Hereinafter, the present invention will be described in more detail with reference to the following examples and experimental examples.

**<Examples>**

**<Example 1> Preparation of crude extracts of *Lindera obtusiloba***

Branches and leaves of *Lindera obtusiloba* collected from Gangwon-do district, South Korea were washed with water to remove foreign substances and salt, and then, dried and pulverized. 25 g of branches or leaves of *Lindera obtusiloba* and a total of 500 mL of 70% by weight of aqueous ethanol solution were added to an extraction vessel, and heated and extracted three times at 70 °C for 3 h under reflux, and filtered with filter papers. The residues were condensed under reduced pressure in a water bath of 40°C and freeze-dried to obtain 6.4 g of a crude extract of *Lindera obtusiloba* subbranches and 7.0 g of a crude extract of *Lindera obtusiloba* leaves.

**<Example 2> Preparation of fractions of crude extracts of *Lindera obtusiloba***

5 g of each crude extract of *Lindera obtusiloba* branches and leaves obtained in Example 1 was suspended in 50 mL of purified water and solvent-fractionation was carried out three times with 50 mL of hexane, ethylacetate, and n-butanol in consecutive order to obtain each solvent fraction. Each solvent fraction was condensed under reduced pressure to obtain hexane fractions, ethylacetate fractions, and n-butanol fractions of *Lindera obtusiloba.*

**<Experimental example 1> Measurement of inhibitory effect of *Lindera obtusiloba* extracts on platelet aggregation**

**1-1. Preparation of experimental animal and washed platelet**

Rats were paralyzed with ethyl ether and blood samples were collected from abdominal aorta with a syringe containing anticoagulant 0.15 M sodium citrate whose volume ratio is 1:9 compared with blood sample volume. Blood samples were centrifuged at 200 x g for 10 min to obtain platelet rich plasma (PRP) as supernatant. PRP was centrifuged at 800 x g for 15 min, and precipitated platelets were washed two times with a washing buffer (137 mM NaCl, 2.9 mM KCl, 1 mM MgCl$_2$, 5 mM glucose, 12 mM NaHCO$_3$, 0.34 mM Na$_2$HPO$_4$, 1 mM EDTA, 20 mM HEPES, 0.25 % BSA, pH 7.4) and suspended in a suspension buffer (suspension buffer. 137 mM NaCl, 2.9 mM KCl, 1 mM MgCl$_2$, 5 mM glucose, 12 mM NaHCO$_3$, 0.34 mM Na$_2$HPO$_4$, 20 mM HEPES, 0.25 % BSA, pH 7.4) to prepare washed platelets. Platelet numbers of washed platelets used in measurement of inhibitory effect on platelet aggregation were counted with a cell counter (cell counter. Hema-vet HV950FS, Drew Scientific, USA), and washed platelets were diluted with a buffer solution so as to adjust the platelet count to 3 x 10$^8$ platelets/mL. Since platelets are aggregated at low temperatures, the foregoing experiment was carried out at room temperature.

**1-2. Measurement of platelet aggregation**

Inhibitory effect on rat platelet aggregation was measured by turbidimetric method using an aggrerometer (Chrono-Log Co., Ltd., Havertown, PA, USA). Washed platelets were incubated at 37°C for 3 min and treated with various concentrations of extracts. After 2 min, platelet aggregation was induced by a platelet aggregation-inducing substance, ADP (22 $\mu$M) or collagen (20 $\mu$g/mL). Platelet aggregation was measured for 10 min and the inhibition extent of platelet aggregation was calculated. Platelets which were not treated with extracts were used for control, and the inhibition extent of platelet aggregation was calculated using the following equation.

[Equation 1]

$$\text{The inhibition extent of platelet aggregation} = [(A - B) / A] \times 100$$

A: aggregation % of control
B: aggregation % of extract-treated platelets

[Table 1]

| Inhibitory effect of *Lindera obtusiloba* extracts on platelet aggregation | | | |
|---|---|---|---|
| Inhibiting agent | | Inhibition (%) | |
| | Final concentration | Collagen | ADP |
| | 0.1 mg/mL | 18.52 | 15.91 |
| Aspirin | 1 mg/ mL | 51.85 | 52.27 |
| | 10 mg/ mL | 94.44 | 93.18 |
| | 0.1 mg/ mL | -7.41 | 15.91 |
| *Lindera obtusiloba* branches | 0.3 mg/ mL | 83.33 | 68.18 |
| | 1 mg/ mL | 87.04 | 81.82 |
| | 0.1 mg/ mL | 7.41 | 9.09 |
| *Lindera obtusiloba* leaves | 0.3 mg/ mL | 57.41 | 54.55 |
| | 1 mg/ mL | 88.89 | 95.45 |

As shown in the above, inhibitory effect of the extract of *Lindera obtusiloba* branches on platelet aggregation induced by collagen was -7.41%, 83.33%, and 87.04% at 0.1 mg/mL, 0.3 mg/mL, and 1 mg/mL, respectively, and IC50 (Inhibitory concentration of 50 %) was 0.28 $\mu$g/mL, very low. Also, inhibitory effect of the extract of *Lindera obtusiloba* branches on platelet aggregation induced by ADP was 15.91%, 68.18%, and 81.82% at 0.1 mg/mL, 0.3 mg/mL, and 1 mg/mL, respectively, and IC50 was 0.26 $\mu$g/mL, very low. Inhibitory effect of the extract of *Lindera obtusiloba* leaves on platelet aggregation induced by collagen was 7.41%, 57.41%, and 88.89% at 0.1 mg/mL, 0.3 mg/mL, and 1 mg/mL, respectively, and IC50 was 0.28 $\mu$g/mL, quite low. Also, inhibitory effect of the extract of *Lindera obtusiloba* leaves on platelet aggregation induced by ADP was 9.09%, 54.55%, and 95.45% at 0.1 mg/mL, 0.3 mg/mL, and 1 mg/mL, respectively, and IC50 was 0.28 $\mu$g/mL, quite low. Overall, extracts of *Lindera obtusiloba* branches and leaves had stronger effect than aspirin which are currently used as a platelet aggregation inhibitor. IC50 value is the concentration that caused 50% inhibition of platelet aggregation, and the smaller the value is, the stronger the inhibitory effect is.

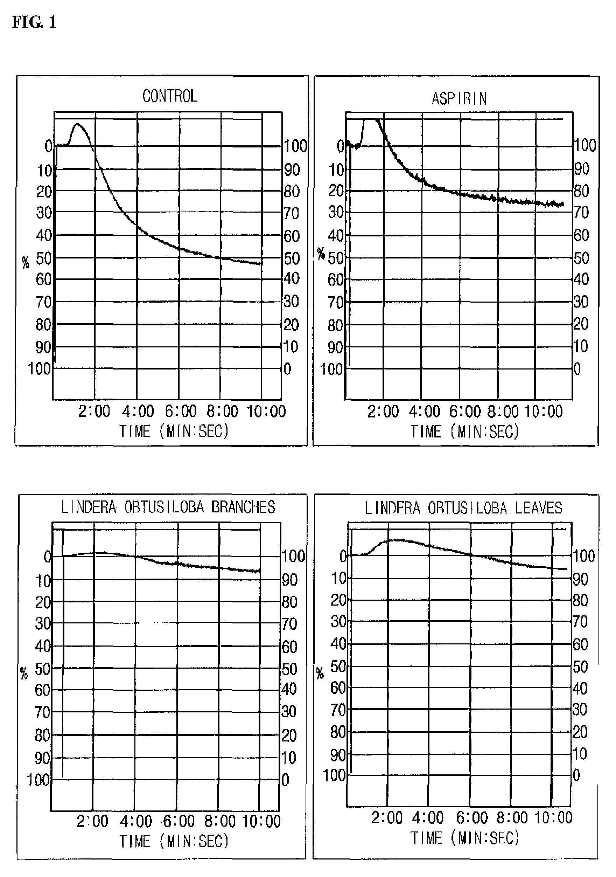

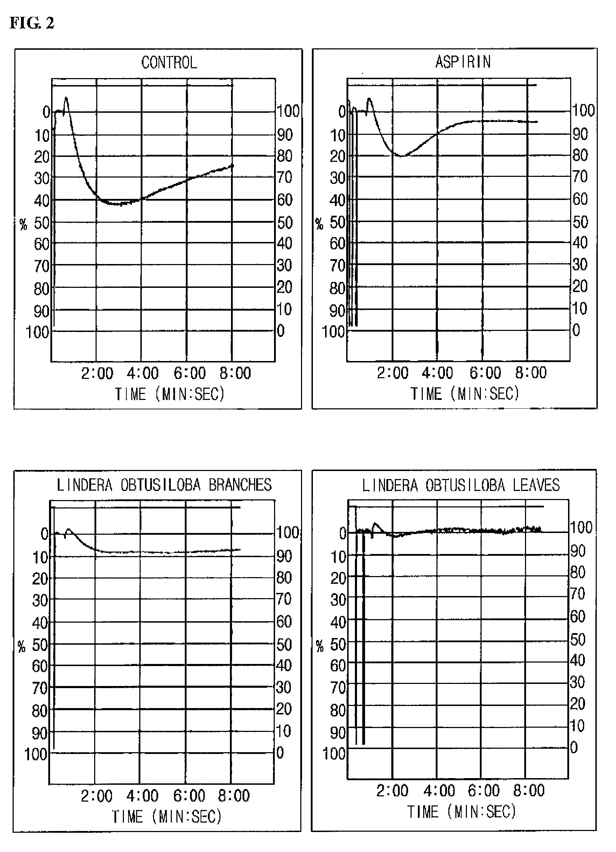

The above result was confirmed also in FIG. 1 and FIG. 2 showing inhibitory effect of extracts of *Lindera obtusiloba* branches and leaves on platelet aggregation induced by collagen or ADP.

**<Experimental example 2> Measurement of inhibitory effect of *Lindera obtusiloba* extracts on thromboembolism**

Experimental thrombosis induction in animals was based on Diminno's method. When a platelet aggregation inducer is injected into a tail vein of a mouse, a large amount of thrombus formation is induced in the pulmonary artery to kill the animal, and by observing whether this can be recovered or not, the inhibition extent of thrombus formation is measured. A mixture solution of collagen (Chrono-Log, 20 $\mu$g/mouse) and epinephrine (Chrono-Log, 2 $\mu$g/mouse) was prepared to be contained in 200 $\mu$L of physiological saline as a platelet aggregation inducer and injected into the tail vein of mice. Extract suspension or physiological saline was orally administered in proportion to body weight at 1h prior to injection of the platelet aggregation inducer to investigate antithrombotic effect. The antithrombotic effect was calculated as percentage of the number of experimental animals protected from hind leg paralysis or death induced by the injection of platelet aggregation inducer. Antithrombotic effect of extracts was determined by observing whether recovery from the persistence of paralysis for 15 min or longer after injection, death, or paralysis occurred or not. The survival rate (%) of each group was calculated with the following equation.

$$[\text{Equation 2}]$$

$$\text{The survival rate (\%)} = (D \, / \, C) \times 100$$

C: the number of animals to which collagen was precisely injected.
D: the number of survived animals among animals to which collagen was precisely injected.

[Table 2]

| Inhibitory effect of *Lindera obtusiloba* extracts on thromboembolism | | | | | |
|---|---|---|---|---|---|
| | 1st | 2nd | 3rd | Sum (D/C) | Survival rate (%) |
| Control | 5/17 | 3/24 | 5/14 | 13/55 | 23 |
| Aspirin | 15/19 | 9/20 | 9/19 | 33/58 | 56 |
| *Lindera obtusiloba* branches | 14/19 | 8/18 | 7/20 | 29/57 | 51 |
| *Lindera obtusiloba* leaves | 13/20 | 4/17 | 10/19 | 27/56 | 48 |

As shown in the above, inhibitory effect of extracts of *Lindera obtusiloba* branches and leaves on thromboembolism was 51% and 48% of the survival rate, respectively. Those were 25 to 30% higher than the survival rate of control. Thus, the result indicated that extracts of *Lindera obtusiloba* branches and leaves have significant inhibitory effect on thromboembolism.

**<Experimental example 3> Cytotoxicity test of *Lindera obtusiloba* extracts**

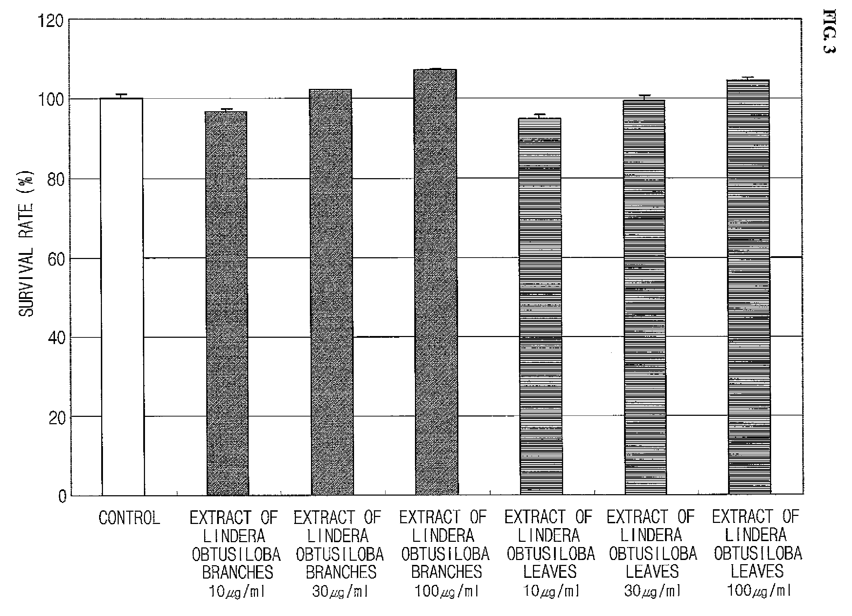

Cytotoxicity of crude extracts of *Lindera obtusiloba* prepared in <Example 1> was compared and the result was shown in FIG. 3.

Vascular smooth muscle cells in artery vessels were mixed with MEM (minimum essential medium) and 10% FBS (fetal bovine serum) solution, and incubated for 24 h at 5% $CO_2$/ 37°C. After cells were stabilized, cells were treated with extracts of *Lindera obtusiloba* obtained in <Example 1> to shake, and incubated for 24 h. Then, MTS solution (cellTiter 96 Aqueous One Solution, promega) was added, and cells were incubated for 1 h, and absorbance was measured at 490 nm.

As shown in FIG. 3, extracts of *Lindera obtusiloba* of the present invention didn't show any effect on cell survival, and thus, the result indicated that extracts of *Lindera obtusiloba* of the present invention are a very safe drug.

**<Experimental example 4> Statistical treatment**

The data were considered significant with a probability less than 0.05 through Student's t-test and one-way ANOVA test for experimental results.

Preparation examples of pharmaceutical compositions and health functional foods comprising the extract of the present invention are described in the following, and these are not to limit the scope of the invention, but to explain the present invention concretely.

**<Preparation example 1> Powder preparation**

> *Lindera obtusiloba* extracts power 20 mg
> Lactose 100 mg
> Talc 10 mg

The above ingredients were mixed, and filled into an airtight bag to prepare a powder.

**<Preparation example 2> Granule preparation**

> *Lindera obtusiloba* extracts power 10 mg
> Corn starch 100 mg
> Lactose 100 mg
> Magnesium stearate 2 mg

The above ingredients were mixed, and tabletted by a conventional tablet preparation method to prepare a tablet.

**<Preparation example 3> Capsule preparation**

> *Lindera obtusiloba* extracts power 10 mg
> Crystalline cellulose 3 mg
> Lactose 14.8 mg
> Magnesium stearate 2 mg

The above ingredients were mixed by a conventional capsule preparation method, and filled in a gelatin capsule to prepare a capsule.

**<Preparation example 4> Injection preparation**

> *Lindera obtusiloba* extracts power 10 mg
> Mannitol 180 mg
> Sterile distilled water for injection 2794 mg

One ample (2 mL) was prepared with the above contents of the ingredients by a conventional injection preparation method.

**<Preparation example 5> Liquid preparation**

*Lindera obtusiloba* extracts power 10 mg
High fructose corn syrup 10 g
Mannitol 5 g
Purified water suitable amount

According to a conventional liquid preparation method, each ingredient was added to purified water and dissolved therein, and lemon flavor was added thereto in a suitable amount. And then, the above ingredients were mixed and purified water added thereto so that the total amount become 100 mL, and filled in a brown bottle and sterilized to prepare a liquid.

**<Preparation example 6> Health beverage preparation**

*Lindera obtusiloba* extracts power 10 mg
Vitamin C 15 g
Vitamin E (powdered) 100 g
Iron lactate 19.75 g
Zinc oxide 3.5 g
Nicotinamide 3.5 g
Vitamin A 0.2 g
Vitamin B1 0.25 g
Vitamin B2 0.3 g
Water suitable amount

The above ingredients were mixed according to a conventional health beverage preparation method, and heated with agitation at 85°C for about 1 h, and then, the prepared solution was filtered to obtain in a sterilized 2L vessel, seal sterilized and refrigeration stored to use for the health beverage composition of the present invention. The above ratios illustrate a preferable example of mixing ingredients relatively suitable for a favorite beverage; however, it can be modified arbitrarily according to regional and ethnic preference such as of the class of consumers or consumer country, the uses, *etc.*

**Claims**

1.  A pharmaceutical composition for use in preventing and treating thrombotic disorders, comprising an extract of leaves of *Lindera obtusiloba* as an active ingredient, wherein the thrombotic disorder is selected from the group of atherothrombotic diseases, phlebothrombosis, hepatic portal vein thrombosis, pulmonary thromboembolism, chronic limb ischemia, varicose vein, deep vein thrombosis diseases, cerebral infarction, and cerebral hemorrhage.

2.  The pharmaceutical composition for use according to claim 1, wherein the extract of leaves of *Lindera obtusiloba* is extracted with a solvent selected from the group consisting of water, a lower alcohol of $C_1$ to $C_5$, and a mixture thereof.

3.  The pharmaceutical composition for use according to claim 2, wherein the extract of leaves of *Lindera obtusiloba* is extracted with from about 30 to about 95 % by weight of an aqueous ethanol solution

4.  The pharmaceutical composition for use according to claim 2, wherein the extract of leaves of *Lindera obtusiloba* is extracted with a solvent selected from the group consisting of water, a lower alcohol of $C_1$ to $C_3$, and a mixture thereof, and then extracted again with butanol.

5.  The pharmaceutical composition for use according to claim 2, wherein the extract of leaves of *Lindera obtusiloba* is prepared by obtaining a crude extract through an extraction with a solvent selected from the group consisting of water, a lower alcohol of $C_1$ to $C_3$, and a mixture thereof and concentration, suspending the crude extract into water, and extracting the suspended crude extract again in the order of hexane, ethylacetate, and butanol.

6. The pharmaceutical composition for use according to claim 1, wherein the extract of leaves of *Lindera obtusiloba* has platelet aggregation-inhibitory activity.

7. The pharmaceutical composition for use according to claim 1, wherein the extract of leaves of *Lindera obtusiloba* has inhibitory and preventive activity on thrombus formation *in vivo.*

8. A health functional food for use in preventing thrombotic disorders, comprising an extract of leaves of *Lindera obtusiloba* as an active ingredient, wherein the thrombotic disorder is selected from the group of atherothrombotic diseases, phlebothrombosis, hepatic portal vein thrombosis, pulmonary thromboembolism, chronic limb ischemia, varicose vein, deep vein thrombosis diseases, cerebral infarction, and cerebral hemorrhage.

**Patentansprüche**

1. Pharmazeutische Zusammensetzung zur Verwendung in der Vorbeugung und Behandlung thrombotischer Erkrankungen, umfassend einen Extrakt von *Lindera obtusiloba* Blättern als Wirkstoff, wobei die thrombotische Erkrankung ausgewählt ist aus der Gruppe von atherothrombotischen Erkrankungen, Phlebothrombose, hepatische Pfortaderthrombose, pulmonale Thromboembolie, chronische Ischämie der Gliedmaßen, Krampfadern, tiefe Venenthrombose-Erkrankungen, Hirninfarkt und Hirnblutung.

2. Pharmazeutische Zusammensetzung zur Verwendung nach Anspruch 1, wobei der Extrakt von *Lindera obtusiloba* Blättern mit einem Lösungsmittel ausgewählt aus der Gruppe, bestehend aus Wasser, einem niederen $C_1$- bis $C_5$-Alkohol und einer Mischung davon, extrahiert ist.

3. Pharmazeutische Zusammensetzung zur Verwendung nach Anspruch 2, wobei der Extrakt von *Lindera obtusiloba* Blättern mit von etwa 30 bis etwa 95 Gewichts% einer wässrigen Ethanollösung extrahiert ist.

4. Pharmazeutische Zusammensetzung zur Verwendung nach Anspruch 2, wobei der Extrakt von *Lindera obtusiloba* Blättern mit einem Lösungsmittel ausgewählt aus der Gruppe, bestehend aus Wasser, einem niederen $C_1$- bis $C_3$-Alkohol und einer Mischung davon, und dann nochmals mit Butanol extrahiert ist.

5. Pharmazeutische Zusammensetzung zur Verwendung nach Anspruch 2, wobei der Extrakt von *Lindera obtusiloba* Blättern hergestellt ist durch Erhalten eines Rohextrakts durch eine Extraktion mit einem Lösungsmittel ausgewählt aus der Gruppe, bestehend aus Wasser, einem niederen $C_1$- bis $C_3$-Alkohol und einer Mischung davon, und Konzentration, Suspendieren des Rohextrakts in Wasser und nochmaligem Extrahieren des suspendierten Rohextrakts in der Reihenfolge von Hexan, Ethylacetat und Butanol.

6. Pharmazeutische Zusammensetzung zur Verwendung nach Anspruch 1, wobei der Extrakt von *Lindera obtusiloba* Blättern Thrombozytenaggregation-hemmende Aktivität aufweist.

7. Pharmazeutische Zusammensetzung zur Verwendung nach Anspruch 1, wobei der Extrakt von *Lindera obtusiloba* Blättern eine hemmende und vorbeugende Aktivität auf *in vivo* Thrombenbildung aufweist.

8. Gesundheitsfunktionslebensmittel zur Verwendung in der Vorbeugung thrombotischer Erkrankungen, umfassend einen Extrakt von *Lindera obtusiloba* Blättern als Wirkstoff, wobei die thrombotische Erkrankung ausgewählt ist aus der Gruppe von atherothrombotischen Erkrankungen, Phlebothrombose, hepatische Pfortaderthrombose, pulmonale Thromboembolie, chronische Ischämie der Gliedmaßen, Krampfadern, tiefe Venenthrombose-Erkrankungen, Hirninfarkt und Hirnblutung.

**Revendications**

1. Une composition pharmaceutique pour usage dans la prévention et le traitement de troubles thrombotiques, comprenant un extrait de feuilles de Lindera obtusiloba en tant qu'un ingrédient actif, dans lequel le trouble thrombotique est choisi parmi le groupe constitué par la maladie athérothrombotique, la phlébothrombose, la thrombose de la veine porte, l'embolie pulmonaire, l'ischémie chronique des membres, les varices, les maladies de thrombose veineuse profonde, l'infarctus cérébral, et l'hémorragie cérébrale.

2. La composition pharmaceutique pour usage selon la revendication 1, dans laquelle l'extrait de feuilles de Lindera obtusiloba est extrait avec un solvant choisi parmi le groupe constitué par l'eau, un alcool inférieur de $C_1$ à $C_5$, et un mélange de ceux-ci.

3. La composition pharmaceutique pour usage selon la revendication 2, dans laquelle l'extrait de feuilles de Lindera obtusiloba est extrait avec d'environ 30 à environ 95 % en poids d'une solution d'éthanol aqueuse.

4. La composition pharmaceutique pour usage selon la revendication 2, dans laquelle l'extrait de feuilles de Lindera obtusiloba est extrait avec un solvant choisi parmi le groupe constitué par l'eau, un alcool inférieur de $C_1$ à $C_3$, et un mélange de ceux-ci, et puis extrait à nouveau avec du butanol.

5. La composition pharmaceutique pour usage selon la revendication 2, dans laquelle l'extrait de feuilles de Lindera obtusiloba est préparé en obtenant un extrait cru par le moyen d'une extraction avec un solvant choisi parmi le groupe constitué par l'eau, un alcool inférieur de $C_1$ à $C_3$, et un mélange de ceux-ci et de la concentration, la suspension de l'extrait cru dans de l'eau, et l'extraction de l'extrait cru suspendu à nouveau dans l'ordre de hexane, acétate d'éthyle, et butanol.

6. La composition pharmaceutique pour usage selon la revendication 1, dans laquelle l'extrait de feuilles de Lindera obtusiloba a une activité d'inhibition d'agrégation plaquettaire.

7. La composition pharmaceutique pour usage selon la revendication 1, dans laquelle l'extrait de feuilles de Lindera obtusiloba a une activité inhibitrice et préventive sur la formation de thrombus in vivo.

8. Un aliment fonctionnel diététique pour usage dans la prévention de troubles thrombotiques, comprenant un extrait de feuilles de Lindera obtusiloba en tant qu'un ingrédient actif, dans lequel le trouble thrombotique est choisi parmi le groupe constitué par la maladie athérothrombotique, la phlébothrombose, la thrombose de la veine porte, l'embolie pulmonaire, l'ischémie chronique des membres, les varices, les maladies de thrombose veineuse profonde, l'infarctus cérébral, et l'hémorragie cérébrale.

FIG. 1

**FIG. 2**

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NESHEIM M.** *Chest.,* September 2003, vol. 124, 33S-9S
- **KWON HAKCHEOL et al.** *Archives of Pharmacal Research,* 1999, vol. 22, 417-422
- *Planta Medica,* 2003, vol. 69, 610-616
- *Archives of Pharmacal Research,* 1999, vol. 22, 417-422
- **HARBORNE. J.B.** Phytochemical methods : A guide to modern techniques of plant analysis. 1998, 6-7